# EUROPÄISCHE PATENTSCHRIFT

(11) **EP 0 635 239 B1**
(45) Veröffentlichungstag und Bekanntmachung des Hinweises auf die Patenterteilung: **15.09.1999**
(21) Anmeldenummer: 94107021.1
(22) Anmeldetag: 05.05.1994
(51) Int. Cl.: A61B 5/0408

(54) **Verfahren und Vorrichtung zur Herstellung von Elektroden**
Method and apparatus for manufacturing electrodes
Méthode et appareil de fabrication d'électrodes

(30) Priorität: 19.07.1993 AT 142193
(43) Veröffentlichungstag der Anmeldung: 25.01.1995
(73) Patentinhaber: Lang, Leonhard, A-6020 Innsbruck (AT)
(72) Erfinder: Lang, Leonhard, A-6020 Innsbruck (AT)
(74) Vertreter: Torggler, Paul, Dr.

(56) Entgegenhaltungen:
- EP-A- 0 142 372
- WO-A-93/00857
- CA-A- 2 107 836
- US-A- 4 063 352

## Beschreibung

Die Erfindung betrifft ein Verfahren zur Herstellung von Elektroden, insbesondere EKG-Elektroden, bei dem auf die Oberseite eines bahnförmigen Trägermaterials Etiketten aufgeklebt werden, wobei Anschlußelemente mit den Etiketten verbunden werden und schließlich aus dem bahnförmigen Trägermaterial mit den hintereinander aufgeklebten Etiketten und den Anschlußelementen einzelne Elektroden mit jeweils einem Etikett und Anschlußelement gebildet, vorzugsweise ausgestanzt werden. Weiters betrifft die Erfindung eine Vorrichtung zur Durchführung eines solchen Verfahrens.

Alle herkömmlichen Verfahren verwenden als Etiketten solche, welche mit einer selbstklebenden Schicht versehen sind. Diese müssen, da selbstklebend, auf einer silikonisierten Trägerschicht zugeführt werden. Um diese Etiketten "spenden" zu können, müssen sie vorher in einem separaten Arbeitsgang angestanzt und das Stanzgitter abgezogen werden. Das Trägerband der Etiketten wird dann in der Elektrodenmaschine um eine scharfe Kante gezogen, dadurch springt das Etikett ab und kann auf die Materialbahn gedrückt werden. Dieses Verfahren hat mehrere wesentliche Nachteile. Die Positionierung auf der Materialbahn kann insbesondere in Längsrichtung nicht ganz genau eingehalten werden. Fehler in dem vorher fertiggestellten Etikettenband werden zu Fehlern auf der Materialbahn. Besonders wichtig aber ist, daß die freiliegende Selbstklebeschicht nicht mehr mit Maschinenteilen in Berührung kommen darf, da diese sonst festklebt. Insbesonders bedeutsam ist dies, wenn der Etikettenbereich inline bedruckt werden soll, da dies eine Gegendruckplatte zur Voraussetzung hat, an welcher das selbstklebende Etikett festkleben würde.

Aufgabe der Erfindung ist es, ein wirtschaftliches Verfahren zur präzisen Herstellung von Elektroden anzugeben. Außerdem soll eine Vorrichtung zur Durchführung dieses Verfahrens geschaffen werden.

Dies wird erfindungsgemäß durch die Merkmale des Anspruchs 1 (Verfahren) bzw. des Anspruchs 17 (Vorrichtung) gelöst.

Als thermoaktivierbare Klebstoffe eignen sich Klebstoffe, die erst bei Wärmeeinwirkung klebrig werden, bei Raumtemperaturen aber nicht selbstklebend sind. Bei Abkühlung auf Normaltemperatur halten diese Klebstoffe zwar die feste Verbindung aufrecht, sind aber nicht mehr klebrig an Stellen, wo keine derartige feste Verbindung aufgebaut wurde. Beispielsweise eignen sich Schmelzklebstoffe (Hotmelt-Klebstoffe), die vorher aus der Schmelze auf die Etikettenbahn aufgebracht werden. Nach dem Aufbringen und Abkühlen der Schmelze sind die so beschichteten Etikettenbahnen bei Raumtemperatur nicht selbstklebend, aber durch Erwärmen thermoaktivierbar. Ein anderer thermoaktivierbarer Kleber kann beispielsweise ein Dispersionskleber sein. Hier wird die Etikettenbahn von der Verwendung im erfindungsgemäßen Verfahren mit einer Dispersion beschichtet. Das Lösungsmittel verdunstet. Zurück bleibt eine Klebstoffschicht auf der Etikettenbahn, die nicht selbstklebend ist, sondern erst durch Wärmeeinwirkung klebrig wird. Grundsätzlich sind auch noch weitere thermoaktivierbare Klebstoffe denkbar und möglich.

Das erfindungsgemäße Verfahren weist zahlreiche Vorteile auf: Die Materialbahn des Trägermaterials liegt genau positioniert unmittelbar unter dem Loch der Etikettenstanze, das ausgestanzte Etikett wird auf beispielsweise 0,1 mm genau auf die erwärmte Materialbahn gepreßt und mit dieser verklebt. Da der thermoaktivierbare Klebstoff sofort wieder auskühlt, ist er (im Gegensatz zu selbstklebenden Etiketten, die immer klebrig sind) in den folgenden Stationen an freiliegenden Stellen nicht mehr klebrig und kann daher ohne Probleme mit Maschinenteilen in Berührung kommen.

Ein weiterer Vorteil ist darin zu sehen, daß die bekannten selbstklebenden Etiketten nur von einem weiteren Hersteller als Ganzes bezogen werden können bzw. nur mit hohem Aufwand selber hergestellt werden können. Durch die Verwendung eines thermoaktivierbaren Klebstoffs können Bahnen, beispielsweise aus PVC oder einem anderen Kunststoff vom Elektrodenhersteller selbst auf kostengünstige Art und Weise mit dem Klebstoff versehen werden. Auch die Lagerhaltung ist vereinfacht, da für verschiedene Etikettengrößen und Formen grundsätzlich dasselbe Band verwendet werden kann, da ja die endgültige Form der Etiketten erst kurz vor dem Aufbringen auf das bahnförmige Trägermaterial, beispielsweise in einer Etikettenstanze festgelegt wird. Damit reicht eine Lagerhaltung von gleichartigen Etikettenbahnen für die Herstellung einer Vielzahl verschiedener Etiketten aus.

Besonders vorteihaft kann durch die Verwendung von bei Raumtemperaturen nicht klebrigen, thermoaktivierbaren Klebstoffen auch ein Bedrucken der Etiketten in der Vorrichtung für die Herstellung der Elektroden selbst vorgenommen werden. Es braucht also nur ein einfarbiges (beispielsweise weißes) Band zugeführt werden, aus dem dann die Etiketten gestanzt und auf das bahnförmige Trägermaterial aufgebracht werden. Danach kühlen die Etiketten sofort aus und können daher leicht bedruckt werden, wobei die für einen Druck im allgemeinen nötige Unterlage, welche mit dem Klebstoff durch ein Loch im bahnförmigen Trägermaterial in Berührung kommt, nicht auf unerwünschte Weise mit den Etiketten verklebt, wie dies beispielsweise von selbstklebenden Etiketten der Fall wäre. Durch das Bedrucken der Etiketten in der Maschine selbst wird die Lagerhaltung weiters vereinfacht, da nicht verschiedenartig bedruckte Etiketten auf Lager gehalten werden müssen, sondern eben nur ein neutrales Band, das an seiner Unterseite mit einem thermoaktivierbaren Klebstoff versehen ist. Die den Kundenwünschen leicht anpaßbare Bedruckung erfolgt dann in der Maschine selbst, nachdem die Etiketten bereits auf das bahnförmige Trägermaterial aufgebracht worden sind.

Für das Aufbringen der Etiketten auf das bahnförmige Trägermaterial bestehen mehrere Möglichkeiten. Zunächst besteht die Möglichkeit vorgefertigte Einzeletiketten zu verwenden, die an ihrer Unterseite mit thermoaktivierbarem Klebstoff beschichtet sind. Um die automatisierte Zufuhr der Etiketten zu erleichtern, ist es besonders günstig, wenn die Etiketten in Form einer durchgehenden, unten mit thermoaktivierbarem Klebstoff beschichteten Bahn zugeführt werden. Es gibt dann bei einer solchen Bahnzufuhr zwei Möglichkeiten. Entweder man bildet aus der Bahn kurz vor dem Aufkleben auf das Trägermaterial einzelne Etiketten (beispielsweise durch Stanzen) und klebt dann diese Einzeletiketten auf. Es gibt aber auch die Möglichkeit, die Etikettenbahn als Ganzes aufzukleben und die Etiketten erst später zu bilden, insbesondere dann, wenn das Trägermaterial zur Bildung der einzelnen Elektroden abgeschnitten bzw. ausgestanzt wird. Gleichzeitig mit diesem Schneide- oder Stanzprozess kann dann auch die auf dem Trägermaterial aufgeklebte Etikettenbahn mitgeschnitten bzw. mitgestanzt werden.

Die Erfindung betrifft auch eine Vorrichtung zur Durchführung des Verfahrens mit einer Führungs- und Fördervorrichtung für das bahnförmige Trägermaterial, welche gekennzeichnet ist durch eine Etikettenaufbringevorrichtung zum Bilden, vorzugsweise Ausstanzen von Etiketten aus einem mit thermoaktivierbarem Klebstoff beschichteten Band und zum Aufbringen derselben auf das Trägermaterial, wobei im Bereich der Etikettenaufbringevorrichtung eine Heizeinrichtung zum Erwärmen des Klebstoffes angeordnet ist.

Weitere Vorteile und Einzelheiten der Erfindung werden in der nachstehenden Figurenbeschreibung näher erläutert.

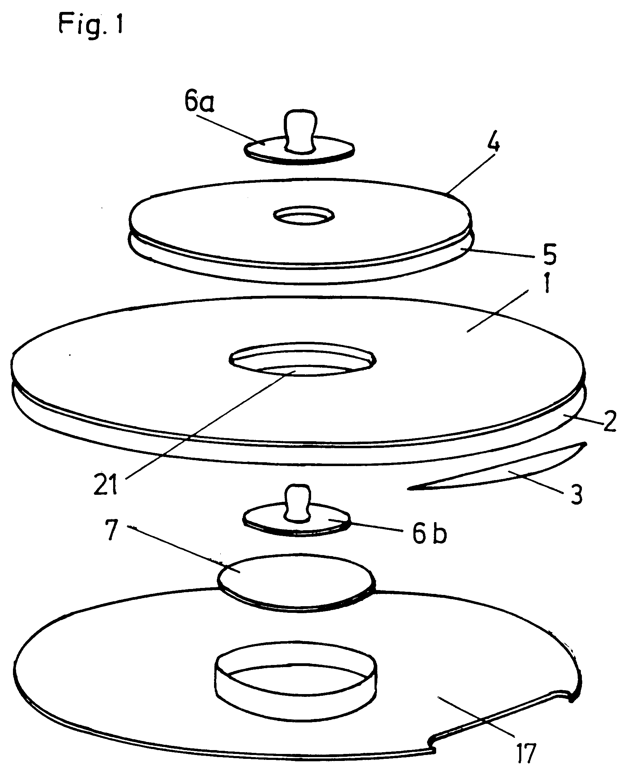

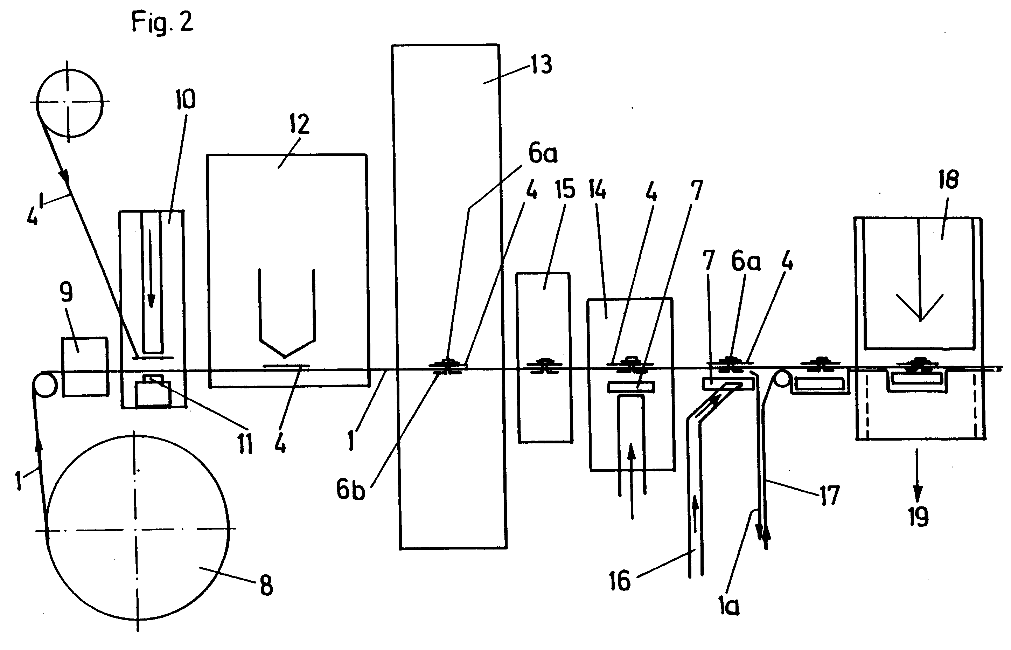

Die Fig. 1 zeigt in einer Explosionsdarstellung eine Elektrode, die mit dem erfindungsgemäßen Verfahren wirtschaftlich und präzise herstellbar ist. Die Fig. 2 zeigt ein Ausführungsbeispiel einer Vorrichtung zur Durchführung des erfindungsgemäßen Verfahrens.

Die in Fig. 1 in einer Explosionsdarstellung dargestellte Elektrode weist ein Trägermaterial 1, beispielsweise aus Schaumstoff, Stoff oder Vlies auf. Das scheibenförmige Trägermaterial weist auf seiner Unterseite eine Selbstklebeschicht 2 auf, die am Rand durch eine kleine Abdeckung abgedeckt ist. Diese kleine Abdeckung 3 erlaubt ein leichteres Abziehen einer Abdeckung 17, die die Selbstklebeschicht 2 vor dem Gebrauch der Elektrode abdeckt und die knapp vor dem Aufkleben der Elektrode auf die Haut des Patienten abgezogen wird, um die Selbstklebeschicht 2 freizulegen.

Das scheibenförmige Trägermaterial weist in der Mitte ein kreisförmiges Loch 21 auf. Dieses Loch ist von oben durch ein Etikett 4, beispielsweise aus PVC oder einem anderen Kunstsoff abgedeckt. Das Etikett 4 ist beim Ausführungsbeispiel über einen Schmelzklebstoff 5 mit dem Trägermaterial 1 verbunden. Das Etikett 4 kann auf seiner Oberseite vorteilhaft bedruckt sein. Es dient vor allem zur lagesicheren Halterung eines Anschlußelementes, das hier aus einer metallischen Niete besteht. Das Nietenoberteil 6a und das Nietenunterteil 6b sind durch das Etikett hindurch miteinander vernietet und damit lagesicher befestigt. Unterhalb der Niete befindet sich ein Schwamm 7, der mit dem Schmelzklebstoff 5 verklebt ist. Der Schwamm 7 ist in an sich bekannter Weise zur elektrischen Potentialübertragung mit einem leitenden Gel getränkt.

In Fig. 2 ist ein Ausführungsbeispiel zur Durchführung des erfindungsgemäßen Verfahrens zur Herstellung von Elektroden, insbesondere EKG-Elektroden gezeigt. Das von einer Rolle 8 abgerollte bahnförmige Trägermaterial wird in Fig. 2 von links nach rechts durch die Vorrichtung bewegt. Es erfolgt ein taktweiser Transport, wobei an sich bekannte Führungs- und Fördervorrichtungen der Übersichtlichkeit halber nicht dargestellt sind. Das Trägermaterial besteht beispielsweise aus einem 35 bis 80 mm breiten selbstklebend beschichteten Schaumstoff, Vliesstoff oder dergleichen, dessen untenliegende Selbstklebeschicht mit silikonisiertem Papier abgedeckt ist. In einer Lochstanze 9 wird mittig ein beispielsweise 15 bis 25 mm großes kreisförmiges Loch aus dem bahnförmigen Trägermaterial ausgestanzt.

Gemäß dem Verfahren der Erfindung wird nun eine mit thermoaktivierbarem Klebstoff, beispielsweise Schmelzklebstoff beschichtete kontinuierliche Bahn 4' einer Etikettenaufbringevorrichtung 10 zugeführt. Beim gezeigten Ausführungsbeispiel ist diese Etikettenaufbringevorrichtung als Stanzvorrichtung 10 ausgebildet, die nacheinander auf der Bahn 4' Etiketten 4 ausstanzt und in einem Arbeitsgang an das Trägermaterial anpreßt. Damit eine Verklebung stattfindet, ist im Bereich der Etikettenaufbringevorrichtung 10 eine Erwärmung des Schmelzklebstoffes an der Unterseite der Etiketten nötig, da dieser sonst bei Raumtemperaturen nicht klebrig ist. Dazu ist eine Heizeinrichtung 11 vorgesehen, die das bahnförmige Trägermaterial 1 von unten erwärmt. Durch die Hitze verklebt der Schmelzklebstoff (Hotmelt) innig mit dem bahnförmigen Trägermaterial. Durch das Ausstanzen und darauffolgende Anpressen ist eine äußerst exakte Positionierung des Etiketts auf dem bahnförmigen Trägermaterial möglich. In der nachfolgenden Tampondruckstation 12 wird das mit dem bahnförmigen Trägermaterial 1 verklebte Etikett bedruckt. Von besonderem Vorteil ist dabei, daß durch geeigneten Abstand der Druckstation 12 von der Etikettenaufbringevorrichtung 10 und eine geeignete Transportgeschwindigkeit des bahnförmigen Trägermaterials erreicht werden kann, daß der Schmelzklebstoff in der Druckstation 12 nicht mehr klebrig ist. Damit können auch die freiliegenden Klebstoffbereiche nicht mit einer Unterlage verkleben, die erst ein Drucken von oben erlaubt.

Beim herkömmlichen Verfahren müssen die Etikettenbahnen mit den verschiedensten Kundendrucken in größerer Menge vorrätig gehalten werden, da pro Kundenauftrag immer eine größere Menge Etiketten vorhanden sein muß, um allfälligen Ausschuß ersetzen zu können. Beim erfindungsgemäßen Verfahren kann der Druck inline erfolgen, die Etikettenmenge entspricht genau der Auftragsmenge, bei Änderung eines Auftrages wird nicht die Etikettenbahn gewechselt, sondern nur das Druckklischee.

In der darauffolgenden Nietstation 13 werden die Nietenteile 6a und 6b durch das Etikett 4 hindurch zusammengenietet und bilden dann das elektrische Anschlußelement. Auch hier ist es von großem Vorteil, daß die Schmelzklebstoffschicht nicht klebrig ist. Als nächstes soll nun in einer Schwammstanze 14 von unten ein Schwamm 7 mit dem Etikett 4 verbunden werden. Da der Schmelzklebstoff bei Raumtemperatur nicht klebrig ist, ist der Schwammstanze 14 eine Heizstation 15 vorgeschaltet, welche mittels Heißluft die freiliegenden Stellen des Schmelzklebstoffs an der Unterseite der Etiketten 4 erwärmt und damit zur Verklebung aktiviert. Durch den geringen Abstand zwischen den Stationen 15 und 14 bleibt die Erwärmung beispielsweise über einem Takt der Maschine erhalten, sodaß ein zuverlässiges Festkleben des Schwämmchens 7 am Etikett 4 möglich ist.

Darauf folgt in an sich bekannter Weise über eine Gelzufuhr 16 eine Tränkung des Schwämmchens 7, das nun mit dem Etikett 4 verklebt ist. Nun ist die Elektrode an sich fertig. Es wird darauf die silikonisierte Papierfolie 1a von der Unterseite des bahnförmigen Materials abgezogen und die damit freiliegende Selbstklebeschicht durch eine silikonisierte Materialbahn, welche bereits die Näpfchen zur Aufnahme des getränkten Schwämmchens 7 beinhaltet, zugeführt. Diese Materialbahn trägt das Bezugszeichen 17.

Zum Schluß werden in der Fergtigstanze 18 die fertigen Elektroden aus dem bahnförmigen Trägermaterial ausgestanzt. Sie fallen in Richtung des Pfeiles 19 aus der Vorrichtung.

Anstelle des oben beschriebenen Schmelzklebstoffes können auch andere thermoaktivierbare Klebstoffe, insbesondere Dispersionsklebstoffe eingesetzt werden, die bei Raumtemperatur nicht selbstklebend sind, bei Wärmeeinwirkung aber klebrig werden.

## Patentansprüche

1. Verfahren zur Herstellung von Elektroden, insbesondere EKG-Elektroden, bei dem auf die Oberseite eines bahnförmigen Trägermaterials (1) Etiketten (4) aufgeklebt werden, wobei Anschlußelemente (6a, 6b) mit den Etiketten (4) verbunden werden und schließlich aus dem bahnförmigen Trägermaterial (1) mit den hintereinander aufgeklebten Etiketten (4) und den Anschlußelementen (6a, 6b) einzelne Elektroden mit jeweils einem Etikett (4) und Anschlußelement (6a, 6b) gebildet, vorzugsweise ausgestanzt werden, dadurch gekennzeichnet, daß mit thermoaktivierbarem Klebstoff (5) beschichtete Etiketten (4) einer Etikettenaufbringevorrichtung (10) zugeführt werden, in welcher die Etiketten (4) jeweils unter Erwärmung des thermoaktivierbaren Klebstoffs (5) an der dem Trägermaterial (1) zugewandten Etikettenunterseite auf das Trägermaterial (1) aufgebracht werden.

2. Verfahren nach Anspruch 1, dadurch gekennzeichnet, daß die Etiketten (4) als mit thermoaktivierbarem Klebstoff (5) beschichtete Einzeletiketten der Etikettenaufbringevorrichtung (10) zugeführt werden.

3. Verfahren nach Anspruch 1, dadurch gekennzeichnet, daß die Etiketten (4) in Form einer kontinuierlichen, mit thermoaktivierbarem Klebstoff (5) beschichteten Bahn (4') der Etikettenaufbringevorrichtung (10) zugeführt werden.

4. Verfahren nach Anspruch 3, dadurch gekennzeichnet, daß in der Etikettenaufbringevorrichtung (10) aus der kontinuierlichen Bahn (4') nacheinander einzelne Etiketten (4) aus der Bahn gebildet, vorzugsweise ausgestanzt werden und diese einzelnen Etiketten (4) auf das Trägermaterial (1) aufgeklebt werden.

5. Verfahren nach Anspruch 4, dadurch gekennzeichnet, daß die Etiketten (4) aus der Bahn (4') mittels eines Stanzstempels ausgestanzt und durch Fortsetzung des Stanzhubes mittels desselben Stanzstempels an das Trägermaterial (1) angepreßt werden.

6. Verfahren nach Anspruch 3, dadurch gekennzeichnet, daß die Etikettenbahn (4') als solche zunächst auf das Trägermaterial (1) aufgeklebt wird und die einzelnen Etiketten (4) später durch gleichzeitiges Zerteilen von Trägermaterial (1) und aufgeklebter Etikettenbahn (4') gebildet werden.

7. Verfahren nach einem der Ansprüche 1 bis 6, dadurch gekennzeichnet, daß das bahnförmige Trägermaterial (1) bei der Etikettenaufbringevorrichtung (10), vorzugsweise von der den Etiketten (4) abgewandten Unterseite her, erwärmt wird.

8. Verfahren nach einem der Ansprüche 1 bis 7, dadurch gekennzeichnet, daß vor der Etikettenaufbringevorrichtung (10) Löcher (21) aus dem bahnförmigen Trägermaterial (1) ausgestanzt werden, wobei jedes Loch (21) in der Etikettenaufbringevorrichtung (10) mit einer im Durchmesser größeren Etikette (4) bzw. Etikettenbahn (4') abgedeckt wird.

9. Verfahren nach einem der Ansprüche 1 bis 8, dadurch gekennzeichnet, daß der Abstand der auf die Etikettenaufbringevorrichtung (10) folgenden Bearbeitungs- bzw. Montagestation sowie die Transportgeschwindigkeit des bahnförmigen Trägermaterials (1) derart gewählt ist, daß der thermoaktivierbare Klebstoff (5) in dieser folgenden Bearbeitungs- bzw. Montagestation durch die inzwischen erfolgte Abkühlung nicht mehr klebrig ist.

10. Verfahren nach einem der Ansprüche 1 bis 9, dadurch gekennzeichnet, daß die auf dem bahnförmigen Trägermaterial (1) aufgeklebten Etiketten (4) bzw. Etikettenbahn (4') bedruckt werden.

11. Verfahren nach einem der Ansprüche 1 bis 10, dadurch gekennzeichnet, daß die Anschlußelemente (6a, 6b) durch metallische Nieten gebildet werden, wobei ein Nietenoberteil (6a) von oben und Nietenunterteil (6b) von unten an das Etikett (4) herangeführt werden, worauf Nietenoberteil (6a) und Nietenunterteil (6b) durch das Etikett (4) hindurch miteinander vernietet werden.

12. Verfahren nach einem der Ansprüche 1 bis 11 dadurch gekennzeichnet, daß auf einem freiliegenden Bereich der Etikettenunterseite ein Schwamm (7) aufgeklebt wird, wobei der thermoaktivierbare Klebstoff (5) knapp vor und/oder beim Aufbringen des Schwammes (7) vorzugsweise mittels Heißluft erwärmt wird.

13. Verfahren nach Anspruch 12, dadurch gekennzeichnet, daß der mit dem Anschlußelement (6a, 6b) in Kontakt stehende Schwamm (7) in an sich bekannter Weise mit einem elektrisch leitfähigen Gel gefüllt wird.

14. Verfahren nach einem der Ansprüche 1 bis 13, dadurch gekennzeichnet, daß die fertigen Elektroden aus dem bahnförmigen Trägermaterial (1), vorzugsweise nach Aufbringen einer abziehbaren Abdeckung (17) auf deren selbstklebenden Unterseite, gestanzt oder geschnitten werden.

15. Verfahren nach einem der Ansprüche 1 bis 13, dadurch gekennzeichnet, daß als thermoaktivierbarer Klebstoff (5) ein Schmelzklebstoff (Hotmelt-Klebstoff) verwendet wird.

16. Verfahren nach einem der Ansprüche 1 bis 14 dadurch gekennzeichnet, daß als thermoaktivierbarer Klebstoff (5) ein Dispersionsklebstoff verwendet wird.

17. Vorrichtung zur Durchführung des Verfahrens nach einem der Ansprüche 1 bis 16, mit einer Führungs- und Fördervorrichtung für ein bandförmige Trägermaterial (1), gekennzeichnet durch eine Etikettenaufbringevorrichtung (10) zum Aufbringen von mit thermoaktivierbarem Klebstoff beschichteten Etiketten (4) auf das Trägermaterial (1), wobei im Bereich der Etikettenaufbringevorrichtung (10) eine Heizeinrichtung (11) zum Erwärmen des Klebstoffes (5) angeordnet ist.

18. Vorrichtung nach Anspruch 17, dadurch gekennzeichnet, daß die Etikettenaufbringevorrichtung (10) aus einer kontinuierlichen, mit thermoaktivierbarem Kleber (5) beschichteteten Bahn (4') einzelne Etiketten (4) bildet, vorzugsweise ausstanzt, und diese dann auf das Trägermaterial (1) aufklebt.

19. Vorrichtung nach Anspruch 17 oder 18, gekennzeichnet durch eine der Etikettenaufbringevorrichtung (10) nachgeschalteten Druckstation (12) zum Bedrucken der auf dem Trägermaterial (1) aufgeklebten Etiketten (4).

20. Vorrichtung nach einem der Ansprüche 17 bis 19, gekennzeichnet durch eine Einrichtung (14) zum Ankleben eines Schwammes (7) auf einen freiliegenden Klebstoff-Bereich (5) an der Etikettenunterseite, wobei diese Einrichtung eine Heizeinrichtung (15), vorzugsweise Heißluft-Heizeinrichtung, zum Erwärmen des freiliegenden Klebstoff-Bereiches (5) umfaßt bzw. knapp vorgeschaltet hat.

## Claims

1. A process for producing electrodes, in particular ECG-electrodes, wherein stickers (4) are glued on to the top side of a carrier material (1) in web form, wherein connecting elements (6a, 6b) are connected to the stickers (4) and finally individual electrodes each having a sticker (4) and connecting element (6a, 6b) are formed, preferably by being stamped out, from the carrier material (1) in web form with the stickers (4) successively glued thereon and the connecting elements (6a, 6b), characterised in that stickers (4) coated with heat-activatable adhesive (5) are fed to a sticker-applying apparatus (10) in which the stickers (4) are respectively applied to the carrier material with heating of the heat-activatable adhesive (5) at the underside of the sticker which is towards the carrier material (1).

2. A process according to claim 1 characterised in that the stickers (4) are fed to the sticker-applying apparatus (10) in the form of individual stickers which are coated with heat-activatable adhesive (5).

3. A process according to claim 1 characterised in that the stickers are fed to the sticker-applying apparatus (10) in the form of a continuous web (4') which is coated with heat-activatable adhesive (5).

4. A process according to claim 3 characterised in that in the sticker-applying apparatus (10) individual stickers (4) are successively formed from the continuous web (4'), preferably by being stamped out of the web, and those individual stickers (4) are glued on to the carrier material (1).

5. A process according to claim 4 characterised in that the stickers are stamped out of the web (4') by means of a stamping die and are pressed against the carrier material (1) by means of the same stamping die by continuing the stamping stroke movement.

6. A process according to claim 3 characterised in that the sticker web (4') is as such firstly glued on to the carrier material (1) and the individual stickers (4) are later formed by simultaneously dividing the carrier material (1) and the glued-on sticker web (4').

7. A process according to one of claims 1 to 6 characterised in that the carrier material (1) in web form is heated in the sticker-applying apparatus (10), preferably from the underside which is remote from the stickers (4).

8. A process according to one of claims 1 to 7 characterised in that upstream of the sticker-applying apparatus (10) holes (21) are stamped out of the carrier material (1) in web form, each hole (21) being covered in the sticker-applying apparatus (10) by a larger-diameter sticker (4) or sticker web (4').

9. A process according to one of claims 1 to 8 characterised in that the spacing of the processing or mounting station which follows the sticker-applying apparatus (10) and the speed of transportation movement of the carrier material (1) in web form are so selected that the heat-activatable adhesive (5) is no longer sticky in that following processing or mounting station due to the cooling effect which has occurred in the meantime.

10. A process according to one of claims 1 to 9 characterised in that the sticker web (4') or stickers (4) which are stuck on to the carrier material (1) in web form are printed upon.

11. A process according to one of claims 1 to 10 characterised in that the connecting elements (6a, 6b) are formed by metal rivets, wherein an upper rivet portion (6a) is moved to the sticker (4) from above and a lower rivet portion (6b) is moved to the sticker (4) from below, whereupon the upper rivet portion (6a) and the lower rivet portion (6b) are riveted together through the sticker (4).

12. A process according to one of claims 1 to 11 characterised in that a sponge (7) is glued on an exposed region of the underside of the sticker, wherein the heat-activatable adhesive (5) is heated, preferably by means of hot air, just before and/or upon application of the sponge (7).

13. A process according to claim 12 characterised in that the sponge (7) which is in contact with the connecting element (6a, 6b) is filled in per se known manner with an electrically conductive gel.

14. A process according to one of claims 1 to 13 characterised in that the finished electrodes are stamped or cut out of the carrier material (1) in web form, preferably after the application of a removable covering (17) to the self-adhesive underside thereof.

15. A process according to one of claims 1 to 13 characterised in that the heat-activatable adhesive (5) used is a hot melt adhesive.

16. A process according to one of claims 1 to 14 characterised in that a dispersion adhesive is used as the heat-activatable adhesive (5).

17. Apparatus for carrying out the process according to one of claims 1 to 16 having a guide and conveyor device for a carrier material (1) in strip form, characterised by a sticker-applying apparatus (10) for applying to the carrier material (1) stickers (4) which are coated with heat-activatable adhesive, wherein a heating device (11) for heating the adhesive (5) is arranged in the region of the sticker-applying apparatus (10).

18. Apparatus according to claim 17 characterised in that the sticker-applying apparatus (10) forms individual stickers (4), preferably by stamping, from a continuous web (4') coated with heat-activatable adhesive (5), and then sticks them on to the carrier material (1).

19. Apparatus according to claim 17 or claim 18 characterised by a printing station (12) disposed downstream of the sticker-applying apparatus (10) for printing on the stickers (4) which are stuck on the carrier material (1).

20. Apparatus according to one of claims 17 to 19 characterised by a device (14) for sticking a sponge (7) on an exposed adhesive region (5) at the underside of the sticker, wherein said device includes or has disposed just upstream thereof a heating device (15), preferably a hot air heating device, for heating the exposed adhesive region (5).

## Revendications

1. Procédé de fabrication d'électrodes, en particulier d'électrodes EKG pour électrocardiogramme, dans lequel des étiquettes (4) sont appliquées par collage sur la face supérieure d'un matériau de support (1) en forme de bande, des éléments de raccordement (6a, 6b) étant reliés aux étiquettes (4), et, enfin, des électrodes individuelles, ayant chacune une étiquette (4) et un élément de raccordement (6a, 6b), étant constituées, de préférence par un processus de découpage par estampage, à partir du matériau de support (1) en forme de bande, avec les étiquettes (4), appliquées par collage les unes derrière les autres, et les éléments de raccordement (6a, 6b), caractérisé en ce que les étiquettes (4), revêtues d'un adhésif (5) thermoactivable, sont amenées à un dispositif d'application d'étiquettes (10) dans lequel les étiquettes (4) sont chacune appliquées sur le matériau de support (1), avec chauffage de l'adhésif (5) thermoactivable placé sur la face inférieure d'étiquette, tournée vers le matériau de support (1).

2. Procédé selon la revendication 1, caractérisé en ce que les étiquettes (4) sont amenées au dispositif d'application d'étiquettes (10) sous forme d'étiquettes individuelles revêtues d'adhésif (5) thermoactivable.

3. Procédé selon la revendication 1, caractérisé en ce que les étiquettes (4) sont amenées au dispositif d'application d'étiquettes (10) sous la forme d'une bande (4') continue, revêtue d'un adhésif (5) thermoactivable.

4. Procédé selon la revendication 3, caractérisé en ce que les étiquettes individuelles (4) sont constituées à partir de la bande, les unes après les autres, dans le dispositif d'application d'étiquettes (10), en partant de la bande (4') continue, de préférence par un processus de découpage par estampage, et ces étiquettes (4) individuelles étant appliquées par collage sur le matériau de support (1).

5. Procédé selon la revendication 4, caractérisé en ce que les étiquettes (4) sont découpées par estampage à partir de la bande (4') au moyen d'un poinçon d'estampage et sont appliquées par pressage par une continuation de la course de déplacement d'estampage, au moyen du même poinçon d'estampage, sur le matériau de support (1).

6. Procédé selon la revendication 3, caractérisé en ce que la bande à étiquettes (4') est appliquée par collage, en tant que telle, d'abord sur le matériau de support (1), et les étiquettes (4) individuelles sont ultérieurement constituées en procédant à une subdivision simultanée du matériau de support (1) et de la bande à étiquettes (4') appliquée par collage.

7. Procédé selon l'une des revendications 1 à 6, caractérisé en ce que, dans le dispositif d'application d'étiquettes (10), le matériau de support (1) en forme de bande est chauffé, de préférence, depuis la face inférieure opposée aux étiquettes (4).

8. Procédé selon l'une des revendications 1 à 7, caractérisé en ce que, en amont du dispositif d'application d'étiquettes (10), des trous (21) sont perforés par estampage à partir du matériau de support (1) en forme de bande, chaque trou (21) étant recouvert, dans le dispositif d'application d'étiquettes (10), par une étiquette (4) ou une bande à étiquettes (4') de dimension supérieure.

9. Procédé selon l'une des revendications 1 à 8, caractérisé en ce que l'espacement entre le poste d'usinage et/ou de montage et le dispositif d'application d'étiquettes (10) en aval duquel il est installé, ainsi que la vitesse de transport du matériau de support (1) en forme de bande sont choisis de manière que, une fois arrivé dans ce poste d'usinage et/ou de montage, l'adhésif (5) thermoactivable ne soit plus collant, suite au refroidissement s'étant effectué dans l'intervalle.

10. Procédé selon l'une des revendications 1 à 9, caractérisé en ce que les étiquettes et/ou la bande d'étiquettes (4') appliquées par collage sur le matériau de support (1) en forme de bande sont imprimées.

11. Procédé selon l'une des revendications 1 à 10, caractérisé en ce que les éléments de raccordement (6a, 6b) sont constitués par des rivets métalliques, une partie supérieure de rivet (6a) étant amenée de façon guidée par le haut et une partie inférieure de rivet (6b) étant amenée de façon guidée par le bas sur l'étiquette (4), suite à quoi la partie supérieure de rivet (6a) et la partie inférieure de rivet (6b) sont rivetées ensemble en traversant l'étiquette (4).

12. Procédé selon l'une des revendications 1 à 11, caractérisé en ce que, sur une zone laissée libre, de la face inférieure d'étiquette, est appliquée, par collage, une éponge (7), l'adhésif (5) thermoactivable étant chauffé, de préférence au moyen d'air chaud, juste avant et/ou lors de l'application de l'éponge (7).

13. Procédé selon la revendication 12, caractérisé en ce que l'éponge (7) mise en contact avec l'élément de raccordement (6a, 6b) est remplie de manière connue en soi d'un gel conducteur de l'électricité.

14. Procédé selon l'une des revendications 1 à 13, caractérisé en ce que les électrodes terminées sont estampées et/ou découpées à partir du matériau de support (1) en forme de bande, de préférence après application d'un revêtement (17) extractible, sur sa face inférieure auto-adhésive.

15. Procédé selon l'une des revendications 1 à 13, caractérisé en ce qu'on utilise, comme adhésif (5) thermoactivable, un adhésif à fusion (adhésif Hotmelt).

16. Procédé selon l'une des revendications 1 à 14, caractérisé en ce qu'on utilise, comme adhésif (5) thermoactivable, un adhésif à dispersion.

17. Dispositif de mise en oeuvre du procédé selon l'une des revendications 1 à 16, avec un dispositif de guidage et de transport pour un matériau de support (1) en forme de bande, caractérisé par un dispositif d'application d'étiquettes (10) destiné à appliquer sur le matériau de support (1) des étiquettes (4) revêtues d'adhésif thermoactivable, dans la zone du dispositif d'application d'étiquettes (10) étant disposé un dispositif de chauffage (11) destiné à chauffer l'adhésif (5).

18. Dispositif selon la revendication 17, caractérisé en ce que le dispositif d'application d'étiquettes (10) est constitué, à partir d'une bande (4') continue, revêtue d'adhésif (5) thermoactivable, d'étiquettes (4) individuelles, de préférence par découpage par estampage, et les applique ensuite sur le matériau de support (1).

19. Dispositif selon la revendication 17 ou 18, caractérisé par un poste d'impression (12) installé en aval du dispositif d'application d'étiquettes (10), pour imprimer les étiquettes (4) appliquées par collage sur le matériau de support (1).

20. Dispositif selon l'une des revendications 17 à 19, caractérisé par un dispositif (14) destiné à appliquer par collage une éponge (7) sur une zone d'adhésif (5) laissée libre sur la face inférieure de l'étiquette, ce dispositif comprenant un, et/ou étant installé juste à la suite d'un dispositif de chauffage (15), de préférence un dispositif de chauffage à l'air chaud, pour chauffer la zone d'adhésif (5) laissée libre.
